# EUROPÄISCHE PATENTSCHRIFT

(11) **EP 1 307 482 B1**
(45) Veröffentlichungstag und Bekanntmachung des Hinweises auf die Patenterteilung: **19.10.2005**
(21) Anmeldenummer: 01962914.6
(22) Anmeldetag: 07.08.2001
(51) Int. Cl.: C07K 14/435

(54) **ZWEIFARBIGES FLUORIMETRISCHES PROTEASEASSAY**
TWO COLOURED FLUORIMETRIC PROTEASE ASSAY
TEST FLUOROMETRIQUE BICHROME SERVANT A DETERMINER L'ACTIVITE DE PROTEASES

(30) Priorität: 07.08.2000 DE 10038382
(43) Veröffentlichungstag der Anmeldung: 07.05.2003
(73) Patentinhaber: Direvo Biotech AG, 50829 Köln (DE)
(72) Erfinder: KUHLEMANN, René, c/o Direvo Biotech AG, 50829 Köln (DE); KOLTERMANN, Andre, c/o Direvo Biotech AG, 50829 Köln (DE); KETTLING, Ulrich, c/o Direvo Biotech AG, 50829 Köln (DE); SCHWILLE, Petra, 37073 Göttingen (DE)
(74) Vertreter: Helbing, Jörg
(86) Internationale Anmeldenummer: PCT/EP2001/009112
(87) Internationale Veröffentlichungsnummer: WO 2002/012543

(56) Entgegenhaltungen:
- ROMOSER, V.A. ET AL: "Detection in living cells of Ca2+ -dependent changes in the fluorescence emission of an indicator composed of two green fluorescent protein variants linked by a calmodulin-binding sequence" THE JOURNAL OF BIOLOGICAL CHEMISTRY, Bd. 272, Nr. 20, 16. Mai 1997 (1997-05-16), Seiten 13270-13274, XP002058387
- KETTLING ULRICH ET AL: "Real-time enzyme kinetics monitored by dual-color fluorescence cross-correlation spectroscopy." PROCEEDINGS OF THE NATIONAL ACADEMY OF SCIENCES OF THE UNITED STATES, Bd. 95, Nr. 4, 17. Februar 1998 (1998-02-17), Seiten 1416-1420, XP001061798 Feb. 17, 1998 ISSN: 0027-8424
- KOLTERMANN ANDRE ET AL: "Rapid assay processing by integration of dual-color fluorescence cross-correlation spectroscopy: High throughput screening for enzyme activity." PROCEEDINGS OF THE NATIONAL ACADEMY OF SCIENCES OF THE UNITED STATES, Bd. 95, Nr. 4, 17. Februar 1998 (1998-02-17), Seiten 1421-1426, XP001061799 Feb. 17, 1998 ISSN: 0027-8424
- DITTRICH PETRA ET AL: "Accessing molecular dynamics in cells by fluorescence correlation spectroscopy." BIOLOGICAL CHEMISTRY, Bd. 382, Nr. 3, März 2001 (2001-03), Seiten 491-494, XP001062907 ISSN: 1431-6730

### SEQUENZPROTOKOLL

<110> DIREVO Biotech AG
   <120> Zweifarbiges fluorimetrisches Proteaseassay
   <130> 011742wo/JH/ml
<140>
   <141>
<160> 12
<170> PatentIn Ver. 2.1
<210> 1
   <211> 487
   <212> PRT
   <213> Künstliche Sequenz
<220>
   <223> Beschreibung der künstlichen Sequenz: Autofluoreszierendes Fusionsprotein
<400> 1
   Met Thr Met Ile Thr Pro Ser Leu His Ala Met Ala Ser Lys Gly Glu
   1               5                   10                  15
   Glu Leu Phe Thr Gly Val Val Pro Ile Leu Val Glu Leu Asp Gly Asp
               20                  25                  30
   Val Asn Gly His Lys Phe Ser Val Ser Gly Glu Gly Glu Gly Asp Ala
           35                  40                  45
   Thr Tyr Gly Lys Leu Thr Leu Lys Phe Ile Cys Thr Thr Gly Lys Leu
       50                  55                  60
   Pro Val Pro Trp Pro Thr Leu Val Thr Thr Leu Cys Tyr Gly Val Gln
   65                  70                  75                  80
   Cys Phe Ser Arg Tyr Pro Asp His Met Lys Arg His Asp Phe Phe Lys
                   85                  90                  95
   Ser Ala Met Pro Glu Gly Tyr Val Gln Glu Arg Thr Ile Phe Phe Lys
               100                 105                 110
   Asp Asp Gly Asn Tyr Lys Thr Arg Ala Glu Val Lys Phe Glu Gly Asp
           115                 120                 125
   Thr Leu Val Asn Arg Ile Glu Leu Lys Gly Ile Asp Phe Lys Glu Asp
       130                 135                 140
   Gly Asn Ile Leu Gly His Lys Leu Glu Tyr Asn Tyr Asn Ser His Asn
   145                 150                 155                 160
   Val Tyr Ile Met Ala Asp Lys Gln Lys Asn Gly Ile Lys Val Asn Phe
                   165                 170                 175
   Lys Thr Arg His Asn Ile Glu Asp Gly Ser Val Gln Leu Ala Asp His
               180                 185                 190
   Tyr Gln Gln Asn Thr Pro Ile Gly Asp Gly Pro Val Leu Leu Pro Asp
           195                 200                 205
   Asn His Tyr Leu Ser Thr Gln Ser Ala Leu Ser Lys Asp Pro Asn Glu
       210                 215                 220
   Lys Arg Asp His Met Val Leu Leu Glu Phe Val Thr Ala Ala Gly Ile
   225                 230                 235                 240
   Thr His Gly Met Asp Glu Leu Tyr Asn Gln Ser Thr Leu Glu Asp Pro
                   245                 250                 255
   Arg Val Pro Val Ala Thr Met Arg Ser Ser Lys Asn Val Ile Lys Glu
               260                 265                 270
   Phe Met Arg Phe Lys Val Arg Met Glu Gly Thr Val Asn Gly His Glu
           275                 280                 285
   Phe Glu Ile Glu Gly Glu Gly Glu Gly Arg Pro Tyr Glu Gly His Asn
       290                 295                 300
   Thr Val Lys Leu Lys Val Thr Lys Gly Gly Pro Leu Pro Phe Ala Trp
   305                 310                 315                 320
   Asp Ile Leu Ser Pro Gln Phe Gln Tyr Gly Ser Lys Val Tyr Val Lys
                   325                 330                 335
   His Pro Ala Asp Ile Pro Asp Tyr Lys Lys Leu Ser Phe Pro Glu Gly
               340                 345                 350
   Phe Lys Trp Glu Arg Val Met Asn Phe Glu Asp Gly Gly Val Val Thr
           355                 360                 365
   Val Thr Gln Asp Ser Ser Leu Gln Asp Gly Cys Phe Ile Tyr Lys Val
       370                 375                 380
   Lys Phe Ile Gly Val Asn Phe Pro Ser Asp Gly Pro Val Met Gln Lys
   385                 390                 395                 400
   Lys Thr Met Gly Trp Glu Ala Ser Thr Glu Arg Leu Tyr Pro Arg Asp
                   405                 410                 415
   Gly Val Leu Lys Gly Glu Ile His Lys Ala Leu Lys Leu Lys Asp Gly
               420                 425                 430
   Gly His Tyr Leu Val Glu Phe Lys Ser Ile Tyr Met Ala Lys Lys Pro
           435                 440                 445
   Val Gln Leu Pro Gly Tyr Tyr Tyr Val Asp Ser Lys Leu Asp Ile Thr
       450                 455                 460
   Ser His Asn Glu Asp Tyr Thr Ile Val Glu Gln Tyr Glu Arg Thr Glu
   465                 470                 475                 480
   Gly Arg His His Leu Phe Leu
                   485
<210> 2
   <211> 506
   <212> PRT
   <213> Künstliche Sequenz
<220>
   <223> Beschreibung der künstlichen Sequenz: Autofluoreszierendes Fusionsprotein
<400> 2
   Met Thr Met Ile Thr Pro Ser Leu His Ala Met Ala Ser Lys Gly Glu
   1               5                   10                  15
   Glu Leu Phe Thr Gly Val Val Pro Ile Leu Val Glu Leu Asp Gly Asp
               20                  25                  30
   Val Asn Gly His Lys Phe Ser Val Ser Gly Glu Gly Glu Gly Asp Ala
           35                  40                  45
   Thr Tyr Gly Lys Leu Thr Leu Lys Phe Ile Cys Thr Thr Gly Lys Leu
       50                  55                  60
   Pro Val Pro Trp Pro Thr Leu Val Thr Thr Leu Cys Tyr Gly Val Gln
   65                  70                  75                  80
   Cys Phe Ser Arg Tyr Pro Asp His Met Lys Arg His Asp Phe Phe Lys
                   85                  90                  95
   Ser Ala Met Pro Glu Gly Tyr Val Gln Glu Arg Thr Ile Phe Phe Lys
               100                 105                 110
   Asp Asp Gly Asn Tyr Lys Thr Arg Ala Glu Val Lys Phe Glu Gly Asp
           115                 120                 125
   Thr Leu Val Asn Arg Ile Glu Leu Lys Gly Ile Asp Phe Lys Glu Asp
       130                 135                 140
   Gly Asn Ile Leu Gly His Lys Leu Glu Tyr Asn Tyr Asn Ser His Asn
   145                 150                 155                 160
   Val Tyr Ile Met Ala Asp Lys Gln Lys Asn Gly Ile Lys Val Asn Phe
                   165                 170                 175
   Lys Thr Arg His Asn Ile Glu Asp Gly Ser Val Gln Leu Ala Asp His
               180                 185                 190
   Tyr Gln Gln Asn Thr Pro Ile Gly Asp Gly Pro Val Leu Leu Pro Asp
           195                 200                 205
   Asn His Tyr Leu Ser Thr Gln Ser Ala Leu Ser Lys Asp Pro Asn Glu
       210                 215                 220
   Lys Arg Asp His Met Val Leu Leu Glu Phe Val Thr Ala Ala Gly Ile
   225                 230                 235                 240
   Thr His Gly Met Asp Glu Leu Tyr Asn Gln Ser Thr Leu Glu Asp Pro
                   245                 250                 255
   Asp Ile Pro Thr Thr Glu Asn Leu Tyr Phe Gln Ser Gly Thr Val Asp
               260                 265                 270
   Ala Asp Pro Arg Val Pro Val Ala Thr Met Arg Ser Ser Lys Asn Val
           275                 280                 285
   Ile Lys Glu Phe Met Arg Phe Lys Val Arg Met Glu Gly Thr Val Asn
       290                 295                 300
   Gly His Glu Phe Glu Ile Glu Gly Glu Gly Glu Gly Arg Pro Tyr Glu
   305                 310                 315                 320
   Gly His Asn Thr Val Lys Leu Lys Val Thr Lys Gly Gly Pro Leu Pro
                   325                 330                 335
   Phe Ala Trp Asp Ile Leu Ser Pro Gln Phe Gln Tyr Gly Ser Lys Val
                   340                     345                 350
   Tyr Val Lys His Pro Ala Asp Ile Pro Asp Tyr Lys Lys Leu Ser Phe
           355                     360                 365
   Pro Glu Gly Phe Lys Trp Glu Arg Val Met Asn Phe Glu Asp Gly Gly
       370                     375                 380
   Val Val Thr Val Thr Gln Asp Ser Ser Leu Gln Asp Gly Cys Phe Ile
   385                 390                     395                 400
   Tyr Lys Val Lys Phe Ile Gly Val Asn Phe Pro Ser Asp Gly Pro Val
                   405                     410                 415
   Met Gln Lys Lys Thr Met Gly Trp Glu Ala Ser Thr Glu Arg Leu Tyr
               420                     425                 430
   Pro Arg Asp Gly Val Leu Lys Gly Glu Ile His Lys Ala Leu Lys Leu
           435                     440                 445
   Lys Asp Gly Gly His Tyr Leu Val Glu Phe Lys Ser Ile Tyr Met Ala
       450                     455                 460
   Lys Lys Pro Val Gln Leu Pro Gly Tyr Tyr Tyr Val Asp Ser Lys Leu
   465                 470                     475                 480
   Asp Ile Thr Ser His Asn Glu Asp Tyr Thr Ile Val Glu Gln Tyr Glu
                   485                     490                 495
   Arg Thr Glu Gly Arg His His Leu Phe Leu
               500                     505
<210> 3
   <211> 547
   <212> PRT
   <213> Künstliche Sequenz
<220>
   <223> Beschreibung der künstlichen Sequenz: Autofluoreszierendes Fusionsprotein
<400> 3
   Met Thr Met Ile Thr Pro Ser Leu His Ala Met Ala Ser Lys Gly Glu
    1               5                   10                  15
   Glu Leu Phe Thr Gly Val Val Pro Ile Leu Val Glu Leu Asp Gly Asp
               20                  25                  30
   Val Asn Gly His Lys Phe Ser Val Ser Gly Glu Gly Glu Gly Asp Ala
           35                  40                  45
   Thr Tyr Gly Lys Leu Thr Leu Lys Phe Ile Cys Thr Thr Gly Lys Leu
       50                  55                  60
   Pro Val Pro Trp Pro Thr Leu Val Thr Thr Leu Cys Tyr Gly Val Gln
   65                  70                  75                  80
   Cys Phe Ser Arg Tyr Pro Asp His Met Lys Arg His Asp Phe Phe Lys
                   85                  90                  95
   Ser Ala Met Pro Glu Gly Tyr Val Gln Glu Arg Thr Ile Phe Phe Lys
               100                 105                 110
   Asp Asp Gly Asn Tyr Lys Thr Arg Ala Glu Val Lys Phe Glu Gly Asp
   115 120 125
   Thr Leu Val Asn Arg Ile Glu Leu Lys Gly Ile Asp Phe Lys Glu Asp
   130 135 140
   Gly Asn Ile Leu Gly His Lys Leu Glu Tyr Asn Tyr Asn Ser His Asn
   145 150 155 160
   Val Tyr Ile Met Ala Asp Lys Gln Lys Asn Gly Ile Lys Val Asn Phe
   165 170 175
   Lys Thr Arg His Asn Ile Glu Asp Gly Ser Val Gln Leu Ala Asp His
   180 185 190
   Tyr Gln Gln Asn Thr Pro Ile Gly Asp Gly Pro Val Leu Leu Pro Asp
   195 200 205
   Asn His Tyr Leu Ser Thr Gln Ser Ala Leu Ser Lys Asp Pro Asn Glu
   210 215 220
   Lys Arg Asp His Met Val Leu Leu Glu Phe Val Thr Ala Ala Gly Ile
   225 230 235 240
   Thr His Gly Met Asp Glu Leu Tyr Asn Gln Ser Thr Leu Glu Asp Pro
   245 250 255
   Thr Ser Gln His Gly Thr Asn Ser Glu Ile Glu Glu Tyr Leu Lys Val
   260 265 270
   Leu Tyr Asp Tyr Asp Ile Pro Thr Thr Glu Asn Leu Tyr Phe Gln Ser
   275 280 285
   Gly Thr Val Asp Ala Gly Ala Asp Ala Gly Lys Lys Lys Asp Gln Lys
   290 295 300
   Asp Asp Lys Val Ala Glu Gln Ala Ser Lys Asp Pro Arg Val Pro Val
   305 310 315 320
   Ala Thr Met Arg Ser Ser Lys Asn Val Ile Lys Glu Phe Met Arg Phe
   325 330 335
   Lys Val Arg Met Glu Gly Thr Val Asn Gly His Glu Phe Glu Ile Glu
   340 345 350
   Gly Glu Gly Glu Gly Arg Pro Tyr Glu Gly His Asn Thr Val Lys Leu
   355 360 365
   Lys Val Thr Lys Gly Gly Pro Leu Pro Phe Ala Trp Asp Ile Leu Ser
   370 375 380
   Pro Gln Phe Gln Tyr Gly Ser Lys Val Tyr Val Lys His Pro Ala Asp
   385 390 395 400
   Ile Pro Asp Tyr Lys Lys Leu Ser Phe Pro Glu Gly Phe Lys Trp Glu
   405 410 415
   Arg Val Met Asn Phe Glu Asp Gly Gly Val Val Thr Val Thr Gln Asp
   420 425 430
   Ser Ser Leu Gln Asp Gly Cys Phe Ile Tyr Lys Val Lys Phe Ile Gly
   435 440 445
   Val Asn Phe Pro Ser Asp Gly Pro Val Met Gln Lys Lys Thr Met Gly
   450 455 460
   Trp Glu Ala Ser Thr Glu Arg Leu Tyr Pro Arg Asp Gly Val Leu Lys
   465 470 475 480
   Gly Glu Ile His Lys Ala Leu Lys Leu Lys Asp Gly Gly His Tyr Leu
   485 490 495
   Val Glu Phe Lys Ser Ile Tyr Met Ala Lys Lys Pro Val Gln Leu Pro
   500 505 510
   Gly Tyr Tyr Tyr Val Asp Ser Lys Leu Asp Ile Thr Ser His Asn Glu
   515 520 525
   Asp Tyr Thr Ile Val Glu Gln Tyr Glu Arg Thr Glu Gly Arg His His
   530 535 540
   Leu Phe Leu
   545
<210> 4
   <211> 32
   <212> PRT
   <213> Künstliche Sequenz
<220>
   <223> Beschreibung der künstlichen Sequenz: Schnittstellensegment
<400> 4
   Gln Ser Thr Leu Glu Asp Pro Asp Ile Pro Thr Thr Glu Asn Leu Tyr
   1 5 10 15
   Phe Gln Ser Gly Thr Val Asp Ala Asp Pro Arg Val Pro Val Ala Thr
   20 25 30
<210> 5
   <211> 7
   <212> PRT
   <213> Künstliche Sequenz
<220>
   <223> Beschreibung der künstlichen Sequenz: Protease-Schnittstelle
<400> 5
   Glu Asn Leu Tyr Phe Gln Ser
   1 5
<210> 6
   <211> 4
   <212> PRT
   <213> Künstliche Sequenz
<220>
   <223> Beschreibung der künstlichen Sequenz: Protease-Schnittstelle
<400> 6
   Ile Glu Gly Arg
   1
<210> 7
   <211> 4
   <212> PRT
   <213> Künstliche Sequenz
<220>
   <223> Beschreibung der künstlichen Sequenz: Protease-Schnittstelle
<400> 7
   Lys Xaa Tyr Lys
   1
<210> 8
   <211> 9
   <212> PRT
   <213> Künstliche Sequenz
<220>
   <223> Beschreibung der künstlichen Sequenz: Protease-Schnittstelle
<400> 8
   Val Gly Pro Arg Ser Phe Leu Leu Lys
   1 5
<210> 9
   <211> 7
   <212> PRT
   <213> Künstliche Sequenz
<220>
   <223> Beschreibung der künstlichen Sequenz: Protease-Schnittstelle
<400> 9
   Gly Lys Tyr Ala Pro Trp Val
   1 5
<210> 10
   <211> 8
   <212> PRT
   <213> Künstliche Sequenz
<220>
   <223> Beschreibung der künstlichen Sequenz: Protease-Schnittstelle
<400> 10
   Ser Gln Asn Tyr Pro Ile Val Gln
   1 5
<210> 11
   <211> 11
   <212> PRT
   <213> Künstliche Sequenz
<220>
   <223> Beschreibung der künstlichen Sequenz: Protease-Schnittstelle
<400> 11
   Arg Gly Val Val Asn Ala Ser Ser Arg Leu Ala
   1 5 10
<210> 12
   <211> 8
   <212> PRT
   <213> Künstliche Sequenz
<220>
   <223> Beschreibung der künstlichen Sequenz: Protease-Schnittstelle
<400> 12
   Leu Val Leu Ala Ser Ser Ser Phe
   1 5

## Beschreibung

Die vorliegende Erfindung betrifft ein autofluoreszierendes Fusionsprotein, das sich als Proteasesubstrat eignet, eine für dieses Fusionsprotein kodierende Nukleinsäuresequenz sowie ein Verfahren unter Verwendung des Fusionsproteins und/oder der Nukleinsäure-Sequenz in einem zweifarbigen, konfokal-fluorimetrischen Assay für den Nachweis und die Quantifizierung proteolytischer Aktivität in flüssigen Proben oder Zellen.

### Hintergrund der Erfindung

Proteasen sind Enzyme, die die hydrolytische Spaltung von Peptidmolekülen katalysieren. Der Nachweis und die quantitative Bestimmung proteolytischer Aktivität ist für verschiedene Forschungszweige wie auch für die pharmazeutische und biotechnologische Industrie von Bedeutung. Verwendung finden entsprechende, als Proteaseassays bezeichnete Testverfahren bei der Suche nach neuen Enzymen mit proteolytischer Aktivität, bei deren biochemischer Charakterisierung, für die Kontaminationskontrolle in Produktionsanlagen und bei der Suche nach Substanzen mit aktivitätsmodifizierenden Eigenschaften. Einen besonderen Schwerpunkt bildet hier das Hochdurchsatz-Screening nach Wirkstoffen mit Protease-inhibierender Wirkung (Protease-Inhibitoren), z.B. für den Einsatz in der Therapie viraler Infektionen.

In Proteaseassays finden vornehmlich Peptide als Substrate Verwendung. Deren Aminosäuresequenzen erlauben eine optimale Interaktion mit dem aktiven Zentrum der Proteasen. In der Literatur ist eine Reihe geeigneter Methoden zur Messung der Hydrolyse dieser Substrate beschrieben, z.B. elektrophoretische, chromatographische oder spektroskopische Verfahren (Methods Enzymol. (1981) 80:341-361, Methods Enzymol. (1994) 241:70-86, Peptides (1991) 787-789). Die bekannten Verfahren zur Analyse von Proteaseaktivität können prinzipiell in heterogene und homogene Verfahren unterteilt werden. Bei heterogenen Methoden erfolgt die Analyse der Hydrolyseprodukte zeitlich von der Reaktion getrennt (*off-line*), hierbei kommen Verfahren wie SDS-PAGE, Western-Blot, ELISA oder HPLC zum Einsatz (J. Immunol. Methods (1993) 161:151-155). Neben dem Nachteil des zeitlichen Abstands zwischen Reaktion und Erhalt des Messwerts sind diese Verfahren insbesondere durch ihre aufwendige Handhabung gekennzeichnet. Vorteilhaft ist die Möglichkeit, vollständig unmodifizierte Substrate einsetzen zu können. Heterogene Verfahren werden aus diesem Grund vornehmlich für Einzelanalysen und Evaluierungen eingesetzt.

Der Vorteil homogener Analyseverfahren liegt darin, dass der zeitliche Ablauf der Reaktion direkt in Echtzeit (*on-line*) betrachtet werden kann. Ein weiterer Vorteil spektroskopischer Methoden gegenüber anderen Verfahren liegt in der vergleichsweise geringen Substanzmenge, die für die Messung nötig ist. Zu den homogenen Verfahren der ersten Generation gehören spektroskopische Verfahren mit chromogenen bzw. fluorogenen Substraten. Die Messung beruht hier auf einer Veränderung des Absorptionsspektrums eines Chromophors (chromogene Substrate) oder der Fluoreszenz-Emission eines Fluorophors (fluorogene Substrate) als Folge der proteolytischen Spaltung (Biochemistry (1967) 67(6): 1765-1777, Anal. Biochem. (1979) 99(1):53-64). Fluorogene Substrate haben gegenüber chromogenen Substraten in der Regel den Vorteil einer höheren Empfindlichkeit und somit der Möglichkeit, weitaus geringere Konzentrationen erfassen zu können. Chromophore und Fluorophore sind in diesen Substraten in der Regel C-terminal über eine Amidbindung an ein Peptid mit einer für die zu bestimmende Protease spezifischen Aminosäuresequenz gekoppelt.

Diese Substrate der ersten Generation zeichnen sich jedoch durch eine Reihe von Nachteilen aus, die ihre Anwendung einschränken. Zunächst handelt es sich bei den Chromophoren und Fluorophoren meist um vergleichsweise große, aromatische Reste (z.B. p-Nitrophenol, β-Naphtylamide, etc.), die sich in ihrer chemischen Natur stark von den Resten der zwanzig natürlich vorkommenden Aminosäuren unterscheiden. Weiterhin unterscheidet sich die üblicherweise für die Kopplung des Chromophors oder des Fluorophors verwendete Amidbindung deutlich von einer Peptidbindung. Dies reduziert die Enzymselektivität und die Umsatzrate drastisch.
Schließlich muss das Chromophor bzw. das Fluorophor in der Regel direkt C-terminal an die Protease-Schnittstelle gebunden werden, damit es bei der Proteasekatalysierten Hydrolyse freigesetzt wird und so seine spektralen bzw. fluoreszenten Eigenschaften ändert.

Völlig ungeeignet sind diese Substrate somit für die Analyse von Substratspezifitäten auf der Peptidkette in einigem Abstand von der Protease-Schnittstelle, d.h. der sekundären Substratspezifität. Beispielsweise bedarf die Protease aus HIV (Human Immunodeficiency Virus) für die Substraterkennung drei oder vier Aminosäurereste auf beiden Seiten der Schnittstelle. Für entsprechende Assays bedarf es demnach längerer Peptide und der Möglichkeit, die Sequenz innerhalb dieser Peptidkette zu variieren. Für diesen Zweck wurden fluoreszente Proteasesubstrate eingeführt, die einen intramolekularen Energietransfer aufweisen (Castillo, M. J. et al., Anal. Biochem. 95 (1979), 228-235; Gershkovich, A. A., und Kholodovych, V. V., J. Biochem. Biophys. Methods 33 (1996), 135-162). Einerseits kann hierzu das Substratpeptid an einem Ende mit einem Fluorophor (Donor) und am anderen Ende mit einem Quencher-Molekül (Akzeptor, z. B. Dabcyl) versehen werden (Matayoshi, E. D. et al., Science 247 (1990), 954-958; Methods in Enzymology (1994) 241:70-86; Anal. Biochem. (1995) 227:148-155). Im intakten Peptidsubstrat löscht der Quencher die Fluoreszenz des Fluorophors weitgehend. Durch die Spaltungsreaktion werden Quencher und Fluorophor jedoch getrennt, wodurch die nach Anregung des Fluorophors messbare Fluoreszenzemission stark ansteigt. An die Stelle des Quencher-Moleküls kann auch ein zweites Fluorophor treten, dessen Excitationsspektrum mit dem Emissionsspektrum des ersten überlappt, so dass es zu einem Fluoreszenz-Resonanz-Energie-Transfer (FRET, Förster-Energietransfer) kommt. Bei Verfahren, die auf diesen Substraten beruhen, hängt die Signalgüte und damit die Sensitivität des Assays insbesondere vom Abstand zwischen Donor und Akzeptor, vom spektralen Überlapp zwischen Emission des Donors und Absorption des Akzeptors und von der Orientierung der Übergangsdipole ab. Diese drei Parameter begrenzen somit auch die Anwendbarkeit derartiger Proteasesubstrate. Bereits bei oben genanntem HIV-Proteaseassay stellt die minimale Länge von sieben Aminosäureresten eine deutliche Limitierung des Assays dar. Ein entsprechendes Substrat bedarf daher einer sorgfältigen Auswahl von Donor und Akzeptormolekül und einer Synthese, die die sterischen Kriterien berücksichtigt (Wang, G.T. et al., Tetrahedron Letters 31 (1990), 6493).

Alternativ zu derartigen, auf der Messung eines Fluoreszenz-Energie-Transfers zwischen zwei chemischen Gruppen basierenden Proteaseassays sind in der Literatur schließlich Peptidsubstrate für die Anwendung in der zweifarbigen, konfokalen Fluorimetrie bekannt (WO 99/34195; Koltermann, A. et al., in: Fluorescence Correlation Spectroscopy - Theory and Application, Springer-Verlag, Rigler,R., Elson,E. (eds.), (2000)). Bei diesen Substraten ist an den beiden Enden des Peptidstrangs jeweils ein Fluorophor chemisch geknüpft, wobei die beiden Fluorophore spektral unterschiedliche Fluoreszenz-Emissions-Eigenschaften aufweisen. Durch Anwendung konfokal-fluorimetrischer Verfahren wie der zweifarbigen Fluoreszenz-Kreuzkorrelations-Spektroskopie (Dual-color FCS, DE 197 57 740) oder der konfokaten Fluoreszenz-Koinzidenz-Analyse (CFCA, WO 99/34195) ist es möglich, den Anteil an Molekülen in einer Testlösung zu bestimmen, an den beide Fluorophore geknüpft sind. Da einzige Bedingung hierfür die Verknüpfung der Fluorophore über eine chemische Bindung ist, wird so die Bestimmung proteolytischer Aktivität ohne Einschränkung durch oben genannte Randbedingungen möglich: der Abstand und die Orientierung zwischen den Fluorophoren ist je nach Assayanforderung frei wählbar. Durch dieses Assayprinzip ist es im Unterschied zu den zuvor genannten Verfahren möglich, Proteaseassays unter nahezu natürlichen Bedingungen *on-line* zu verfolgen, da ganze Proteindomänen als Schnittstelle verwendet werden können und die räumliche Orientierung der beiden außen angebrachten Fluorophore zueinander unerheblich ist. Außerdem können mit der Methode auch in kleinsten Probenvolumen (bis zu wenigen Pikoliter) noch subnanomolare Konzentrationen zweifarbiger Fluorophore detektiert werden.

Wiederum nachteilig an derartigen, bekannten Proteasesubstraten mit chemisch gekoppelten Fluorophoren ist schließlich die aufwendige Prozedur, diese zu synthetisieren. Peptide können zwar per Festphasensynthese bis zu einer Länge von etwa 50 Aminosäuren relativ effizient synthetisiert werden, die zweifache, ortsspezifische Kopplung von Fluorophoren erfordert jedoch einen erheblichen Aufwand, da die Verwendung findenden Fluorophore in der Regel nicht mit der Peptidsynthese-Chemie kompatibel sind. Und auch nur leicht geänderte Peptidsequenzen, um beispielsweise eine leicht veränderte Substratspezifität zu analysieren, erfordern jeweils den gleichen, hohen Syntheseaufwand. Deutlich einfacher ist die Herstellung von Polypeptiden mittels gentechnischer Verfahren, d.h. durch Konstruktion einer Nukleinsäuresequenz, die die Expression eines von ihr kodierten Polypeptids unter Verwendung zellulärer oder zellfreier Expressionssysteme ermöglicht.

Weiterhin entsprechen alle bislang genannten Substrate nicht den Anforderungen eines intrazellulären Protease-Assays. Viele Proteasen werden von den exprimierenden Zellen nicht in das umgebende Medium ausgeschleust, sondern liegen intrazellulär, bei eukaryontischen Zellen auch spezifisch nur in einzelnen Kompartimenten vor. Ein Zellaufschluss mit dem Ziel, die Proteasen einer Messung zugänglich zu machen, ist in der Regel aufwendig. Außerdem geht die räumliche Auflösung verloren. Moderne konfokale Fluoreszenz-Methoden ermöglichen jedoch gerade die ortsaufgelöste, intrazelluläre Fluoreszenz-Messung (Schwille, P. et al., Biophys. J. 77 (1999), 2251). Um Protease-Aktivitäten intrazellulär per fluorimetrischen Verfahren messen zu können, muss das entsprechende Substrat in die Zelle eingebracht oder dort hergestellt werden. Methoden zum Einbringen von Substanzen sind bekannt, z.B. per Mikroinjektion oder Elektroporation. Allerdings leiden alle diese Verfahren daran, dass sie relativ aufwendig sind, dass sie grundsätzlich zu einer Schädigung der Zelle führen sowie dass eine mögliche Kontrolle von Menge und Ort des Einbringens schwierig ist. Alternativ hierzu kann jedoch mit bekannten molekularbiologischen Verfahren eine Nukleinsäuresequenz, die für das Proteasesubstrat kodiert, in die Zelle eingebracht werden. Die anschließende, ebenfalls extern kontrollierbare Expression der Nukleinsäuresequenz in das korrespondierende Polypeptid ergibt dann das Proteasesubstrat. Derartige Proteasesubstrate sind dann einer Fluoreszenzmessung zugänglich, wenn an den Code für das eigentliche Protease-Substrat derjenige für ein oder mehrere auto-fluoreszente Proteine (AFPs) geknüpft wird.

Die Möglichkeit eines derartigen, vollständig exprimierbaren Proteasesubstrats ist in der Literatur bereits bekannt (Mitra, R.D. et al., Gene 173 (1996), 13 - 93). Diesem Zweck dient eine Nukleinsäuresequenz, die den Code für eine Proteaseschnittstelle zwischen für zwei Varianten des GFP (Green Fluorescent Protein, aus Aequorea victoria) kodierenden Sequenzen enthält. Die GFP-Varianten sind derart gewählt, dass bei intakter Peptidbrücke (beschrieben ist eine Brücke mit 20 Aminosäureresten und einer Schnittstelle für Faktor Xₐ) ein Fluoreszenz-Energie-Transfer zwischen ihnen messbar ist. Proteolytische Aktivität führt zu einer Trennung der beiden Fluorophore und damit zu einem Abfall der Fluoreszenzernission des Akzeptor-Fluorophors. Dieses Verfahren löst zwar die mit chemisch zu synthetisierenden Substraten verbundenden Probleme und ist prinzipiell für intrazelluläre Assays geeignet. Allerdings unterliegt es allen, oben genannten Nachteilen von Fluoreszenz-Energie-Transfer-Substraten, wie beispielsweise der Limitierung im strukturellen Design des Substrats und der maximal möglichen Länge der Protease-Erkennungs-Sequenz.

Der vorliegenden Erfindung liegt somit das technische Problem zugrunde, ein Proteasesubstrat zu entwerfen und ein Verfahren zur Messung proteolytischer Aktivität unter Anwendung dieses Proteasesubstrats zu stellen, welche zusammengenommen die oben beschriebenen Nachteile bekannter Proteasesubstrate und bekannter Verfahren zur Messung proteolytischer Aktivität vermeiden. Insbesondere soll das Substrat für eine Synthese mittels zellulärer oder zellfreier Expressionssysteme geeignet sein und die intrazelluläre Analyse von Proteaseaktivitäten ermöglichen. Es soll die Notwendigkeit der aufwendigen, regiospezifischen chemischen Kopplung von Fluorophoren an Polypeptide vermeiden. Schließlich soll es ein uneingeschränktes Design der Proteaseschnittstelle ermöglichen.

### Zusammenfassung der Erfindung

Überraschenderweise wurde gefunden, dass ein spezielles autofluoreszierendes Fusionsprotein, das zwei unterscheidbare autofluoreszierende Proteine umfasst, die vorstehend ausgeführten Anforderungen erfüllt. Die Erfindung betrifft somit
(1) ein autofluoreszierendes Fusionsprotein, das ein erstes autofluoreszierendes Protein, ein Schnittstellensegment mit einer Protease-Schnittstelle und mindestens ein weiteres, vom ersten unterscheidbares, autofluoreszierendes Protein umfasst, wobei zwischen den beiden autofluoreszierenden Proteinen kein wesentlicher Fluoreszenzenergietransfer stattfindet;
(2) eine Nukleinsäuresequenz, die für ein autofluoreszierendes Fusionsprotein, wie in (1) definiert, kodiert;
(3) einen Vektor, umfassend eine Nukleinsäuresequenz, wie in (2) definiert;
(4) eine Zelle oder einen transgenen Organismus, umfassend die Nukleinsäuresequenz, wie in (2) definiert, und/oder den Vektor, wie in (3) definiert;
(5) ein Verfahren zur Herstellung des autofluoreszierenden Fusionsproteins, wie in (1) definiert, umfassend die Expression der Nukleinsäuresequenz, wie in (2) definiert, mit Hilfe eines zellulären oder zellfreien Expressionssystems;
(6) ein Verfahren zur Analyse einer Probe auf proteolytische Aktivität, bestehend aus den Schritten:
   (a) Zusammenführen des autofluoreszierenden Fusionsproteins, wie in (1) definiert, mit der auf proteolytische Aktivität zu untersuchenden Probe in einer wässrigen Testlösung;
   (b) Inkubation unter Bedingungen, die für eine proteolytische Spaltung geeignet sind; und
   (c) Messung des Anteils an gespaltenem Fusionsprotein mittels konfokal-fluorimetrischer Methoden;
(7) ein Verfahren zur Analyse einer Probe auf protease-inhibierende Aktivität, bestehend aus den Schritten:
   (a) Zusammenführen des autofluoreszierenden Fusionsproteins, wie in (1) definiert, mit der auf protease-inhibierende Aktivität hin zu untersuchende Probe sowie der entsprechende Protease in einer wässrigen Testlösung;
   (b) Inkubation unter Bedingungen, die für eine proteolytische Spaltung geeignet sind; und
   (c) Messung des Anteils an gespaltenem Fusionsprotein mittels konfokal-fluorimetrischer Methoden;
(8) ein Verfahren zur Analyse intrazellulärer Protease-Aktivität, bestehend aus den Schritten:
   (a) Einführen der Nukleinsäuresequenz, wie in (2) definiert, und/oder des Vektors, wie in (3) definiert, in die zu untersuchende Zelle, so dass das autofluoreszierende Fusionsprotein, wie in (1) definiert, intrazellulär exprimiert wird;
   (b) Inkubation unter Bedingungen, die für eine Expression und eine proteolytische Spaltung des Fusionsproteins geeignet sind; und
   (c) Bestimmung der intrazellulär vorliegenden Protease-Aktivität mittels konfokal-fluorimetrischer Methoden; und
(9) ein Verfahren zur Analyse intrazellulärer, protease-inhibierender Aktivität, bestehend aus den Schritten:
   (a) Einführen der Nukleinsäuresequenz, wie in (2) definiert, und/oder des Vektors, wie in (3) definiert, in die zu untersuchende Zelle, so dass das autofluoreszierende Fusionsprotein, wie in (1) definiert, intrazellulär exprimiert wird;
   (b) Inkubation unter Bedingungen, die für eine Expression und eine proteolytische Spaltung des Fusionsproteins geeignet sind; und
   (c) Bestimmung der intrazellulär vorliegenden protease-inhibierende Aktivität mittels konfokal-fluorimetrischer Methoden.

Die Erfindung wird nachfolgend näher erläutert.

### Figurenbeschreibung:

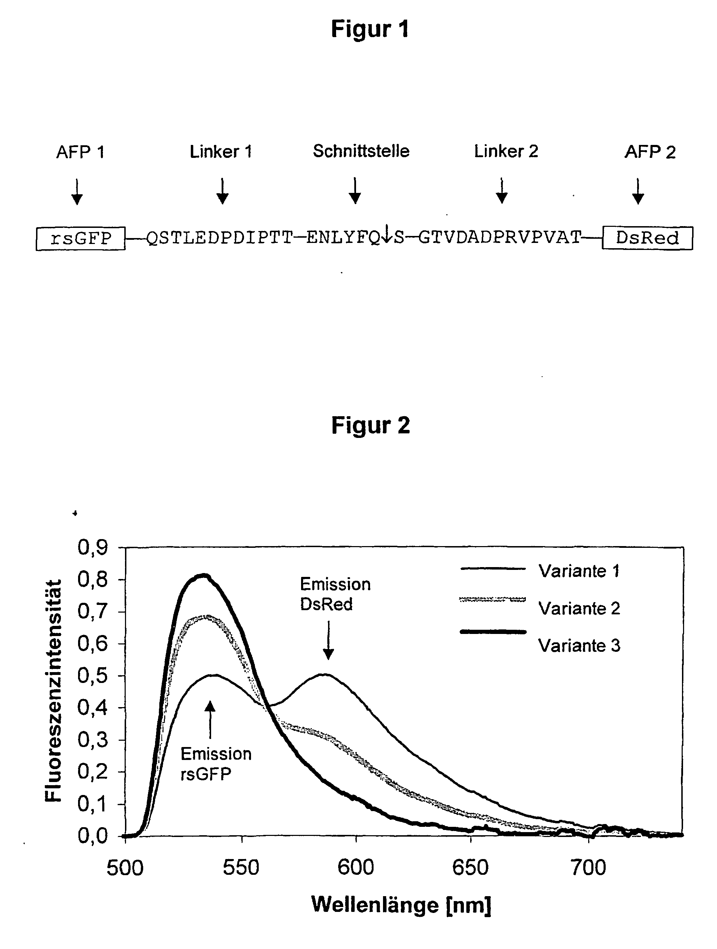

Figur 1: Schematische, beispielhafte Darstellung des erfindungsgemäßen Fusionsproteins B (siehe auch SEQ ID NO:2). Das erste autofluoreszenzte Protein (AFP) ist rsGFP, das zweite DsRed, zwischen den beiden Linkersequenzen befindet sich eine spezifische Schnittstelle für die Protease aus dem Tobacco-Etch-Virus (TEV).
Figur 2: Fluoreszenz-Emissionsspektren von drei Fusionsprotein-Varianten mit der Anregungswellenlänge 488 nm; AFP 1 ist rsGFP, AFP 2 ist DsRed. In Variante 1 (Fusionsprotein A; SEQ ID NO:1) befinden sich zwischen den beiden autofluoreszenten Proteinen 13 Aminosäuren, in Variante 2 (Fusionsprotein B; SEQ ID NO:2) 32 Aminosäuren, in Variante 3 (Fusionsprotein C; SEQ ID NO:3) 73 Aminosäuren. Anhand der sinkenden Emission des DsRed ist deutlich die mit zunehmender Linkerlänge einhergehende Reduktion des FRET zu erkennen. Die erfindungsgemäße Verwendung der Fusionsproteine als Proteasesubstrate und deren Messung mittels Kreuzkorrelationsanalyse oder Koinzidenzanalyse bleibt davon unberührt.

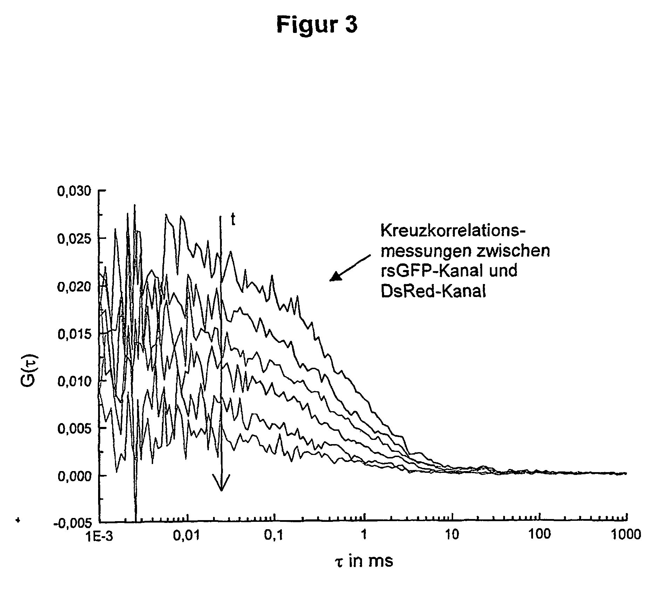

Figur 3: Messung der Zweiphotonen-Kreuzkorreiation während der Spaltung des Fusionsproteins in der Variante 2 (Fusionsprotein B; SEQ ID NO:2) durch Einwirkung der TEV-Protease. Die Spaltung der Bindung und die damit einhergehende Verringerung des Anteils zweifach fluoreszenter Moleküle in der Messlösung bewirkt das Abfallen der Kreuzkorrelationsamplitude.

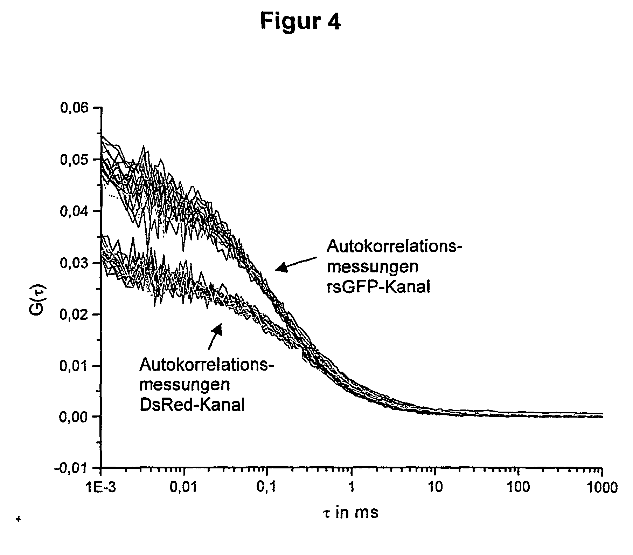

Figur 4: Messung der beiden Zweiphotonen-Autokorrelationen während der Spaltung des Fusionsproteins in der Variante 2 (Fusionsprotein B; SEQ ID NO:2) durch Einwirkung der TEV-Protease. Die Spaltung der Bindung wirkt sich weder auf die Fluoreszenzintensität noch auf die Konzentration oder auf die Teilchengröße der beiden AFPs messbar aus.

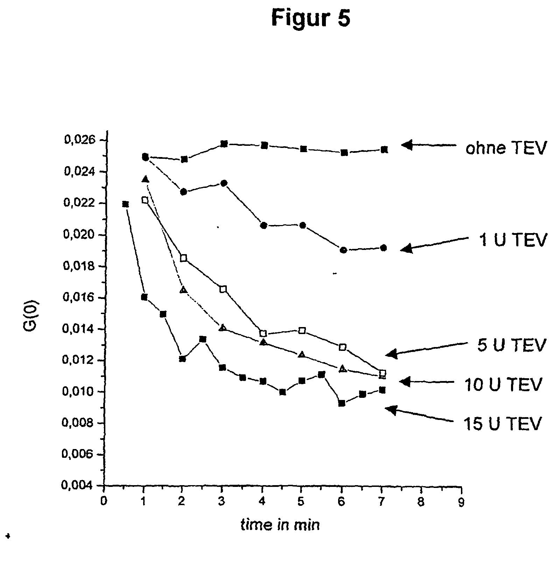

Figur 5: Zeitlicher Verlauf der Zweiphotonen-Kreuzkorrelationsamplitude während der Spaltung des Fusionsproteins in der Variante 2 (Fusionsprotein B; SEQ ID NO:2) durch Einwirkung unterschiedlicher Mengen der TEV-Protease.

### Ausführliche Beschreibung der Erfindung

Das Fusionsprotein gemäß Ausführungsform (1) der Erfindung besteht aus mehreren Teilsequenzen, die zusammen einen durchgehenden Proteinstrang ergeben. Der Proteinstrang enthält erfindungsgemäß ein erstes auto-fluoreszierendes Protein, ein Schnittstellensegment mit einer Protease-Schnittstelle und mindestens ein weiteres, vom ersten unterscheidbares, autofluoreszierendes Protein, wobei zwischen den beiden autofluoreszierenden Proteinen kein wesentlicher Fluoreszenzenergietransfer stattfindet. In einer bevorzugten Ausführungsform ist das Schnittstellensegment in dem Proteinstrang zwischen den zwei autofluoreszierenden, anhand ihrer spektralen Eigenschaften voneinander unterscheidbaren Polypeptiden angeordnet.

"Kein wesentlicher Fluoreszenzenergietransfer" im Sinne der vorliegenden Erfindung bedeutet dabei, dass die emittierte Fluoreszenz der zwei (oder mehr) autofluoreszierenden Proteine nicht oder nur zu weniger als 50 %, vorzugsweise zu weniger als 30 %, besonders bevorzugt zu weniger als 10% das jeweilige andere autofluoreszierende Protein anregt. Das lässt sich zum einen durch den Abstand der beiden Fluoreszenzproteine zueinander, d. h. durch die Länge des Spacerpeptids zwischen den autofluoreszierenden Proteinen (hier Schnittstellensegment) beeinflussen. Andererseits ist aber auch die Art des Spacerproteins, d. h., inwieweit das Spacerpeptid zur Sekundärstrukturbildung (Faltungen) neigt, für den Fluoreszenzenergietransfer relevant. Im Sinne der vorliegenden Erfindung sind daher insbesondere solche Schnittstellensegmente (mit Protease-Schnittstelle) bevorzugt, die keine Sekundärstruktur bilden und relativ starr sind. Die Schnittstellensegmente gemäß der vorliegenden Erfindung weisen insbesondere eine Länge von wenigstens 10, vorzugsweise von mehr als 20, besonders bevorzugt von wenigstens 30 Aminosäureresten, auf.

Erfindungsgemäß handelt es sich bei den autofluoreszierenden Proteinen um Proteine, die nach erfolgter Expression mittels eines zellulären Proteinbiosyntheseapparats oder mittels eines zellfreien Systems zur ribosomalen Proteinsynthese und gegebenenfalls nach anschließender Modifikation durch zelluläre Komponenten oder zugesetzte Enzyme fluoreszente Eigenschaften aufweisen.

"Spektral unterscheidbar" im Sinne der vorliegenden Erfindung sind Fluorophore erfindungsgemäß insbesondere dann, wenn ihre Emissionsspektren voneinander unterscheidbar sind. In einer bevorzugten Ausführung finden als autofluoreszierende Proteine einerseits das Green Fluorescent Protein aus Aequorea victoria oder eine Variante, insbesondere eine Red-Shifted-Variante, davon und andererseits das DsRed aus Discosoma sp. oder eine Variante davon Verwendung. Besonders bevorzugt ist, wenn ein Fusionsprotein mit rsGFP (siehe SEQ ID NO:1, AS 11 bis 249) und DsRed (SEQ ID NO:1, AS 263 bis 487) verwendet wird.

Als Protease-Schnittstellen sind insbesondere solche geeignet, die spezifisch von der Protease aus HIV (Human Immunodeficiency Virus; erkennt die AS-Sequenz SQNYPIVQ), von der Protease aus dem Hepatitis C Virus, von der Protease aus TEV (Tobacco Etch Virus; erkennt die AS-Sequenz ENLYFQS), von der Protease aus hCMV (human Cytomegalovirus; erkennt die AS-Sequenz RGVVNASSRLA), von der Protease aus HSV (Herpes Simplex Virus; erkennt die AS-Sequenz LVLASSSF), von der Protease Plasmin (erkennt die AS-Sequenz KXYK), von der Protease ACE (Angiotensin Converting Enzyme; erkennt die AS-Sequenz GKYAPWV), von der Protease tPA, vom Faktor Xₐ (erkennt die AS-Sequenz IEGR), Thrombin (erkennt die AS-Sequenz VGPRSFLLK) usw. erkannt und gespalten werden.

Besonders bevorzugte Fusionsproteine sind die folgenden Fusionsproteine A - C (das Schnittstellensegment ist unterstrichen, die Proteaseschnittstelle fett gezeichnet; ↓ kennzeichnet die Schnittstelle für die TEV-Protease).

Das Verfahren gemäß Ausführungsform (6) der Erfindung ist ein Verfahren zur Analyse einer Probe auf proteolytische Aktivität und besteht aus den Schritten:
(a) Zusammenführen des mit einem zellulären oder zellfreien Expressionssystem hergestellten erfindungsgemäßen autofluoreszierenden Fusionsproteins mit der auf proteolytische Aktivität zu untersuchenden Probe in einer wässrigen Testlösung;
(b) Inkubation unter Bedingungen, die für eine proteolytische Spaltung geeignet sind;
(c) Messung des Anteils an gespaltenem Fusionsprotein mittels konfokal-fluorimetrischen Methoden.

Eine zu untersuchende Probe kann erfindungsgemäß ein natürliches Isolat, eine chemische Komponente aus einer Substanzbank, eine Fraktion aus einer Fermentationsbrühe oder eine durch Variationstechniken erzeugte Proteaselibrary sein.

Konfokal-fluorimetrische Methoden sind Methoden, die die auf dem Durchtreten einzelner Fluorophore durch einen Anregungsfokus basierenden Fluoreszenzsignale auswerten. Zu den konfokal-fluorimetrische Methoden gehören insbesondere die Fluoreszenz-Korrelations-Spektroskopie (FCS), die zweifarbige Fluoreszenz-Kreuzkorrelations-Spektroskopie (KK-FCS), die konfokale Fluoreszenz-Koinzidenz-Analyse (CFCA) und die 2D-Fluoreszenz-Intensitäts-Verteilungs-Analyse (2D-FIDA).

Gemäß der Ausführungsform (7) betrifft die Erfindung ein Verfahren zur Analyse einer Probe auf protease-inhibierende Aktivität. Dieses Verfahren ist dabei analog zu dem oben genannten Verfahren der Ausführungsform (6), wobei anstelle der auf proteolytische Aktivität zu untersuchenden Probe eine auf protease-inhibierende Aktivität hin zu untersuchende Probe sowie die entsprechende Protease zugesetzt wird.

Unter der entsprechenden Protease ist erfindungsgemäß eine derjenigen Proteasen zu verstehen, die eine mit dem Proteasesubstrat übereinstimmende Sequenzspezifität aufweisen.

Gemäß der Ausführungsform (8) betrifft die Erfindung ein Verfahren zur Analyse intrazellulärer Protease-Aktivität. Dieses Verfahren ist dabei analog zu dem obengenannten Verfahren der Ausführungsform (6), wobei eine für das Fusionsprotein kodierende Nukleinsäure-Sequenz derart in Zellen eingeführt wird, dass sie intrazellulär exprimiert wird, und- anhand der konfokal-fluorimetrischen Messung die intrazellulär vorliegende Protease-Aktivität analysiert werden kann.

Gemäß der Ausführungsform (9) betrifft die Erfindung ein Verfahren zur Analyse intrazellulärer protease-inhibierender Aktivität analog zu den obengenannten Verfahren (6) bis (8), wobei die für das Fusionsprotein und die für die entsprechende Protease kodierenden Nukleinsäure-Sequenzen derart in Zellen eingeführt werden, dass sie intrazellulär exprimiert werden, und anhand der konfokal-fluorimetrischen Messung die intrazellulär vorliegende, protease-inhibierende Aktivität analysiert werden kann.

In einer bevorzugten Ausführungsform erfolgt die Einführung der Nukleinsäure-Sequenz in eukaryotische Zellen anhand eines Vektors, der eine lokal und zeitlich kontrollierte Expression ermöglicht. In einer besonders bevorzugten Ausführungsform erfolgt die Expression nur in ausgewählten Zellkompartimenten.

In einer weiteren besonders bevorzugten Ausführungsform wird als konfokal-fluorimetrische Messmethode die zweifarbige Fluoreszenz-Kreuzkorrelations-Spektroskopie (KK-FCS), die konfokale Fluoreszenz-Koinzidenz-Analyse (CFCA) oder die 2D-Fluoreszenz-Intensitäts-Verteilungs-Analyse (2D-FIDA) eingesetzt.

Erfindungsgemäß kann zusätzlich zu zweifarbigen konfokal-fluorimetrischen Messmethoden als weiterer Messparameter auch der Anteil der einen Fluoreszenz-Energie-Transfer aufweisenden Moleküle bestimmt werden.

In einer weiteren, besonders bevorzugten Ausführungsform wird das Verfahren für die screening-basierende, evolutive Optimierung von Biomolekülen mit proteolytischer Aktivität oder die screening-basierende, evolutive Generierung von Biomolekülen mit proteolytischer Aktivität angewendet.

Die Erfindung wird anhand des nachfolgenden, nicht einschränkenden Beispiels näher erläutert.

### Beispiel

### Konstruktion und Aufreinigung eines Fusionsproteins aus rsGFP und dessen Anwendung in einem Assay für die TEV-Protease

Strategie der Klonierung: Da für FCS-Messungen kleinste Substanzmengen ausreichend sind, wurde zur Expression der Fusionsproteine ein Vektor mit *lac*-Promotor verwendet, womit das Risiko der Bildung von Einschlüssen in den Bakterien (*inclusion bodies)* minimiert wurde. Um eine alternative Aufreinigung mit Hilfe der Nickelchelatchromatographie zu ermöglichen, wurden alle für die Expression in *E. coli* vorgesehenen Fusionsproteine zusätzlich mit einer C-terminalen Hexahistidinsequenz versehen. Da zunächst nicht bekannt war, inwieweit die Aminosäuren im weiteren Umfeld der Erkennurigssequenz der Protease die Reaktion beeinflussen, wurde eine aus dem Polyprotein des *Tobacco-Etch-Virus* stammende Peptidsequenz zum Einfügen zwischen die beiden Fluorphore gewählt. Diese liegt im Bereich der Spaltstelle zwischen Kapsidprotein und Polymerase des Virus. Von der Länge der zwischen den beiden fluoreszenten Proteinteilen liegenden Peptidsequenz hängt unter anderem die Zugänglichkeit der Schnittstelle für die TEV-Protease ab. Deshalb wurden eine kurze Variante mit einem Schnittstellensegment mit 32 Aminosäureresten (Variante 2; STEV; SEQ ID NO:2) und eine lange Variante mit einem Schnittstellensegment mit 73 Aminosäureresten (Variante 3; LTEV; SEQ ID NO:3) hergestellt, in deren Mitte sich jeweils die Schnittstelle befindet. Aus dem TEV-Polyprotein wurden hierfür die Aminosäuren von 2761 bis 2819 für das lange und von 2781 bis 2797 für das kurze Insert ausgewählt.
Im ersten Schritt wurden zwei Konstrukte hergestellt, bei denen jeweils das Gen für *rsGFP* vor dem für *DsRed* eingefügt ist und sich mit diesem im gleichen Leseraster befindet. Für die Expression in *E. coli* wurde zunächst das GFP-Gen vom Vektor *pQBI63* (Qbiogene) amplifiziert. Das PCR-Produkt wurde mit den Restriktionsendonukleasen *Sal I* und *Sph I* verdaut und das Insert anschließend in das ebenso geschnittene Plasmid *pDsRed* (Clontech) eingefügt. Das Ergebnis der Klonierung wurde durch Verdau der Plasmide mit den zuvor verwendeten Restriktionsenzymen überprüft. Die abschließende Sequenzierung ergab, dass die Klonierung erfolgreich und ohne Mutationen verlaufen war ("pGFRed"; Variante 1; SEQ ID NO:1). Im nächsten Schritt wurde das Gen für das Fusionsprotein an seinem 3'-Ende durch eine für sechs aufeinander folgende Histidinreste kodierende Nukleotidsequenz erweitert, um eine spätere Aufreinigung der Proteine durch Nickelchelatchromatographie zu ermöglichen.

Dies geschah analog der oben beschriebenen Vorgehensweise durch Amplifikation des DsRed-Gens vom Plasmid *pDsRed.* Das Insert und das Plasmid *pGFRed* wurden anschließend mit Restriktionsenzymen *Sal I* und *Not I* geschnitten. Das nach der Ligation erhaltene Konstrukt wird im folgenden mit *pGFRed-CH* bezeichnet. Auch hier wurde das Ergebnis der Klonierung durch Verdau der Plasmid-DNA mit den verwendeten Restriktionsenzymen und durch Sequenzierung überprüft.

Die oben beschriebenen Konstrukte (*pGFRed, pGFRed-CH*) besitzen zwischen den Genen der beiden fluoreszenten Proteinteile noch eine kurze, aus dem verbliebenen Rest der 5'-MCS stammende Nukleotidsequenz. Diese Abschnitte wurden im folgenden durch die Insertion synthetisch hergestellter Sequenzen erweitert. Auf diese Weise konnte die Länge der zwischen den beiden fluoreszenten Proteinteilen liegenden Peptidkette variiert und eine spezifische Proteaseschnittstellen in das später gebildete Protein eingefügt werden.

Es wurden zwei Peptidsequenzen unterschiedlicher Länge konzipiert, in deren Mitte sich eine im gesamten Protein einmalige Schnittstelle für die TEV-Protease befindet und die sowohl in das ursprüngliche als auch in das mit der Polyhistidinsequenz versehene GFRed-Protein integriert wurden. Die Sequenzen wurden durch direktes Einfügen einer synthetischen Nukleotidsequenz generiert. Hierfür wurden die Vektoren mit *BamH I* geschnitten und die Phosphatgruppen der freien Enden mit alkalischer Phosphatase entfernt. Die beiden synthetischen Oligonukleotide wurden phosphoryliert, um sie dann als Doppelstrang in die Vektoren einfügen zu können. Die so hergestellten Plasmide sind *pSTev* (Variante 2; SEQ ID NO:2; kurzes Insert), *pSTev-CH* (kurzes Insert mit C-terminaler Hexahistidinsequenz), *pLTev* (Variante 3; SEQ ID NO:3; langes Insert) bzw. *pLTV-CH* (langes Insert mit C-terminaler Hexahistidinsequenz).

Die Genabschnitte können aufgrund der Verwendung von nur einer Schnittstelle jeweils in zwei verschiedenen Orientierungen insertiert werden. Die Orientierung des Inserts wurde durch eine analytische PCR überprüft. Ein PCR-Produkt war nur dann vorhanden, wenn der Genabschnitt in der richtigen Orientierung eingefügt worden war. Das Verfahren selbst lässt sich auch auf andere Restriktionsschnittstellen anwenden. Auch können Art und Position der Proteaseschnittstelle innerhalb des späteren Proteins durch geeignete Wahl der eingefügten Nukleotidsequenz frei gewählt werden.

Proteinexpression in E. coli: Klone mit entsprechendem Vektorkonstrukt wurden aus Dauerkultur auf eine Selektiv-Nähragarplatte ausgestrichen und über Nacht bei 37 °C inkubiert. Eine Kolonie diente zum Animpfen einer Vorkultur von 10 ml Selektiv-Medium. Diese wurde ebenfalls über Nacht bei 37 °C geschüttelt und diente am nächsten Morgen als Inokulum für zwei Ansätze von 200 ml Selektiv-Medium, die mit je 5 ml Vorkultur angeimpft wurden. Wenn die Kulturen eine optische Dichte von 0,7 - 0,8 bei 600 nm erreichten, wurde die Proteinexpression durch Zugabe von jeweils 100 µl IPTG-Lösung (0,5 mM final) induziert. Die Bakterien wurden nach vier Stunden in der Kühlzentrifuge bei 4 °C und 5000 g für 15 Minuten abzentrifugiert und der Überstand verworfen.

Aufschluss der Bakterien: Die Bakterien wurden in 20 ml Puffer resuspendiert, anschließend mit einer Spritze durch eine Nadel mit 0,25 mm Durchmesser gepresst und anschließend in der French Press lysiert. Das Lysat wurde in einem eisgekühlten Zentrifugenröhrchen aufgefangen, in dem bereits 100 µl einer Lösung von 100 mg/ml PMSF in Ethanol zur Inhibition von Proteasen vorgelegt waren. Die Zelltrümmer wurden sofort in der Kühlzentrifuge bei 4 °C und 20000 x g für 20 Minuten abzentrifugiert. Das Bakterienlysat wurde durch einen Spritzenfilter gedrückt und vor dem Auftrag auf die Säule ein Aliquot von 100 µl abgenommen.

Proteinaufreinigung durch Anionenaustauschchromatographie: Als Säulenmaterial wurde DEAE-Sephacel verwendet, der Säulendurchmesser betrug 1 cm und die Füllhöhe 5 cm. Die Säule wurde blasenfrei gepackt und vor dem Beladen mit mindestens 50 ml Waschpuffer equilibriert; die Flussrate betrug 5 ml/min. Die Bakterien wurden im Waschpuffer aufgeschlossen und die Säule mit dem filtrierten Überstand beladen; der Durchlauf wurde zur späteren Analyse aufgefangen.

Dann wurde die Säule mit 40 ml Puffer gewaschen und der Durchfluss ebenfalls aufbewahrt. An das Säulenmaterial gebundenes Protein wurde über einen linearen Gradienten von 50 mM bis 1 M Natriumchlorid in Waschpuffer eluiert und Fraktionen von jeweils 30 Tropfen (etwa 2 ml) aufgefangen; das Gesamtvolumen betrug 100 ml. Abschließend wurde die Säule mit 2 M Natriumchlorid in Waschpuffer regeneriert und anschließend mit 50 ml Waschpuffer equilibriert. Alle Fraktionen wurden bei den Excitationswellenlängen 488 nm und 543 nm auf Fluoreszenz geprüft. Die Fraktionen mit starker Fluoreszenz wurden vereinigt.

Proteinaufreinigung durch Nickelchelatchromatographie: Es wurde eine HiTrap-Chelating-Säule (Pharmacia) mit 1 ml Säulenvolumen verwendet; die Flussrate betrug beim Auftragen des Lysats 0,5 ml/min und sonst 1 ml/min. Die Säule wurde zunächst mit 10 ml Wasser gewaschen und dann mit 2 ml Nickelchloridlösung (100 mM) beladen. Die Entfernung von unspezifisch gebundenen Ni²⁺-Ionen erfolgte durch 10 ml einer Lösung von 300 mM Imidazol in Waschpuffer; anschließend wurde die Säule mit 10 ml Waschpuffer equilibriert. Der filtrierte Überstand der aufgeschlossenen Bakterien wurde auf die Säule aufgetragen, diese wurde anschließend mit 10 ml Puffer gewaschen und das Protein dann mit 8 ml 100 mM Imidazol in Waschpuffer eluiert. Die aufgefangenen Fraktionen von je 1 ml wurden auf Fluoreszenz untersucht und die Säule mit 10 ml 500 mM Imidazol in Puffer gereinigt. Der Durchlauf von Lade-, Wasch- und Reinigungsschritt wurden jeweils aufgefangen und zur späteren Analyse aufbewahrt.

Die zu untersuchenden Proben wurden mit Hilfe einer Centricon-Säule bei 1000 x g aufkonzentriert, mit 100 µl PBS-Puffer versetzt und nochmals bei 1000 x g zentrifugiert. Für die Messung wurden sie so mit PBS-Puffer verdünnt, dass die Meßwerte in einem sinnvollen Bereich liegen.

Fluoreszenzspektren: Von jeder Probe wurden Emissionsspektren bei einer Excitationswellenlänge von 488 nm aufgenommen. In Figur 2 sind die Emissionsspektren (λ_{Ex.}=488 nm) der drei über Anionenaustauschchromatographie aufgereinigten Fusionsproteine *GFRed, STev* und *LTev* dargestellt. Die Spektren sind hierbei jeweils auf die Summe der Fluoreszenzintensitäten bei 540 nm und 580 nm normiert, um die Intensitäten relativ zueinander vergleichen zu können. Die beiden Wellenlängen wurden für die Normierung gewählt, da hier etwa die Maxima von *DsRed* und der *rsGFP* liegen, deren Summe ein ungefähres Maß für die Gesamtintensität darstellt.

Es fällt auf, dass die Graphen der Präparate *GFRed* und *STev* neben dem erwarteten Maximum der GFP-Emission bei etwa 540 nm noch ein weiteres Maximum, bzw. eine Schulter bei etwa 580 nm aufweisen, was im Spektrum des Proteins *LTev* jedoch nicht erkennbar ist. Dieses Phänomen wurde durch Energietransfer (FRET) innerhalb des Proteins verursacht. Dieser Energietransfer liegt im Überlappen des Emissionsspektrums des *rsGFP* mit dem Excitationsspektrum des *DsRed* begründet. Mit zunehmender Linkerlänge reduziert sich der Anteil des Energietransfers.

Fluoreszenzkorreiationsspektroskopische Messungen: Die Messung der proteolytischen Aktivität der TEV-Protease mit den aus *E. coli* präparierten Substratproteinen erfolgte in einer Nunc-Probenkammer mit einem selbst entwickelten FCS-Gerät. Die Substratkonzentrationen betrugen 50 nM. Es wurden jeweils zwei Ansätze von 100 µl Substrat in PBS-Puffer hergestellt, von denen der eine mit der TEV-Protease (verschiedene Konzentrationen) versetzt wurde und der andere als Negativkontrolle diente. Die Messung erfolgte bei Zweiphotonenanregung mit einer Wellenlänge von 950 nm. Detektiert wurde nach farblicher Separation über einen Dichroiten (D530) in den beiden Bereichen 500 nm bis 550 nm (rsGFP-Kanal, Detektionsfilter: 525DF50) und 560 nm bis 610 nm (DsRed-Kanal, Detektionsfilter: 585DF50). Die Messzeit war 60 s, Kinetiken wurden über mehrere Minuten online verfolgt. Die Messungen sind in den Figuren 3 bis 5 dargestellt.

## Patentansprüche

1. Autofluoreszierendes Fusionsprotein, das ein erstes autofluoreszierendes Protein, ein Schnittstellensegment mit einer Protease-Schnittstelle und mindestens ein weiteres, vom ersten unterscheidbares, autofluoreszierendes Protein umfasst, wobei die emittierte Fluoreszenz der zwei (oder mehr) autofluoreszierenden Proteine nicht oder zu weniger als 50 % die jeweiligen anderen autofluoreszierenden Proteine anregt.

2. Autofluoreszierendes Fusionsprotein nach Anspruch 1, wobei das Schnittstellensegment
(i) zwischen zwei autofluoreszierenden, anhand ihrer spektralen Eigenschaften voneinander unterscheidbaren Polypeptiden angeordnet ist und/oder
(ii) neben der Protease-Schnittstelle noch terminale Linkerpeptide aufweist und/oder
(iii) eine Länge von wenigstens 10, vorzugsweise von mehr als 20, besonders bevorzugt von wenigstens 30 Aminosäureresten aufweist.

3. Autofluoreszierendes Fusionsprotein nach Anspruch 1 oder 2, wobei das Schnittstellensegment eine Protease-Schnittstelle aufweist, die spezifisch von der Protease aus dem Human Immunodeficiency Virus, von der Protease aus dem Hepatitis C Virus, von der Protease aus dem Tobacco Etch Virus, von der Protease aus human Cytomegalovirus, von der Protease aus Herpes Simplex Virus, von der Protease Plasmin, von der Protease Angiotensin Converting Enzyme, von der Protease tPA vom Faktor Xₐ und/oder von Thrombin erkannt und gespalten wird.

4. Autofluoreszierendes Fusionsprotein nach einem der Ansprüche 1 bis 3, wobei das erste autofluoreszierende Protein das Green Fluorescent Protein aus Aequorea victoria (GFP) oder eine Variante, insbesondere eine red-shifted-Variante (rsGFP), davon und das zweite autofluoreszierende Protein das dsRed aus Discosoma sp. oder eine Variante davon ist.

5. Autofluoreszierendes Fusionsprotein nach einem der Ansprüche 1 bis 4, das weiterhin funktionale Peptidsequenzen, insbesondere Signalpeptide, Affinitätsmarkerpeptide oder Detektionsmarkerpeptide, aufweist.

6. Autofluoreszierendes Fusionsprotein nach Anspruch 1, das die Sequenz von Fusionsprotein B oder C (SEQ. ID. NOs:2 oder 3) aufweist.

7. Nukleinsäuresequenz, die für ein autofluoreszierendes Fusionsprotein, wie in Ansprüchen 1 bis 6 definiert, kodiert.

8. Vektor, umfassend eine Nukleinsäuresequenz, wie in Anspruch 7 definiert.

9. Zelle oder nicht-humaner transgener Organismus, umfassend die Nukleinsäuresequenz nach Anspruch 7 und/oder den Vektor nach Anspruch 8.

10. Verfahren zur Herstellung des autofluoreszierenden Fusionsproteins nach einem der Ansprüche 1 bis 6, umfassend die Expression der Nukleinsäuresequenz nach Anspruch 7 mit Hilfe eines zellulären oder zellfreien Expressionssystems.

11. Verfahren zur Analyse einer Probe auf proteolytische Aktivität, bestehend aus den Schritten:
(a) Zusammenführen des autofluoreszierenden Fusionsproteins, wie in Ansprüchen 1 bis 6 definiert, mit der auf proteolytische Aktivität zu untersuchenden Probe in einer wässrigen Testlösung;
(b) Inkubation unter Bedingungen, die für eine proteolytische Spaltung geeignet sind; und
(c) Messung des Anteils an gespaltenem Fusionsprotein mittels konfokal-fluorimetrischer Methoden.

12. Verfahren zur Analyse einer Probe auf protease-inhibierende Aktivität, bestehend aus den Schritten:
(a) Zusammenführen des autofluoreszierenden Fusionsproteins, wie in Ansprüchen 1 bis 6 definiert, mit der auf protease-inhibierende Aktivität hin zu untersuchende Probe sowie der entsprechende Protease in einer wässrigen Testlösung;
(b) Inkubation unter Bedingungen, die für eine proteolytische Spaltung geeignet sind; und
(c) Messung des Anteils an gespaltenem Fusionsprotein mittels konfokal-fluorimetrischer Methoden.

13. Verfahren zur Analyse intrazellulärer Protease-Aktivität, bestehend aus den Schritten:
(a) Einführen der Nukleinsäuresequenz nach Anspruch 7 und/oder des Vektors nach Anspruch 8 in die zu untersuchende Zelle, so dass das autofluoreszierende Fusionsprotein, wie in Ansprüchen 1 bis 6 definiert, intrazellulär exprimiert wird;
(b) Inkubation unter Bedingungen, die für eine Expression und eine proteolytische Spaltung des Fusionsproteins geeignet sind; und
(c) Bestimmung der intrazellulär vorliegenden Protease-Aktivität mittels konfokal-fluorimetrischer Methoden.

14. Verfahren zur Analyse intrazellulärer, protease-inhibierender Aktivität, bestehend aus den Schritten:
(a) Einführen der Nukleinsäuresequenz nach Anspruch 7 und/oder des Vektors nach Anspruch 8 in die zu untersuchende Zelle, so dass das autofluoreszierende Fusionsprotein, wie in Ansprüchen 1 bis 6 definiert, intrazellulär exprimiert wird;
(b) Inkubation unter Bedingungen, die für eine Expression und eine proteolytische Spaltung des Fusionsproteins geeignet sind; und
(c) Bestimmung der intrazellulär vorliegenden protease-inhibierende Aktivität mittels konfokal-fluorimetrischer Methoden.

15. Verfahren nach einem der Ansprüche 11 bis 14, wobei als Messmethode eine zweifarbige konfokal-fluorimetrische Messmethode, insbesondere die zweifarbige Fluoreszenz-Kreuzkorrelations-Spektroskopie (KK-FCS), die konfokale Fluoreszenz-Koinzidenz-Analyse (CFCA) oder die 2D-Fluoreszenz-Intensitäts-Verteilungs-Analyse (2D-FIDA) eingesetzt wird.

16. Verfahren nach Anspruch 15, wobei zusätzlich zu zweifarbigen konfokal-fluorimetrischen Messmethoden auch der Anteil der einen Fluoreszenz-Energie-Transfer aufweisenden Moleküle bestimmt wird.

17. Verwendung des Verfahrens nach einem der Ansprüche 11 bis 16 für die screening-basierende, evolutive Optimierung von Biomolekülen mit proteolytischer Aktivität und/oder die screening-basierende, evolutive Generierung von Biomolekülen mit proteolytischer Aktivität.

## Claims

1. An autofluorescent fusion protein which consists of a first autofluorescent protein, a cleavage site segment with a protease cleavage site and at least one further autofluorescent protein distinguishable from the first, whereby the emitted fluorescence of the two (or more) autofluorescent proteins does not or by less than 50 % excite the respective other autofluorescent proteins.

2. Autofluorescent fusion protein according to claim 1, whereby the cleavage site segment
(i) is located between two autofluorescent polypeptides, distinguishable from one another by their spectral properties and/or
(ii) includes terminal linker peptides in addition to the protease cleavage site and/or
(iii) has a length of at least 10, preferably more than 20, most preferably at least 30 amino acid residues.

3. Autofluorescent fusion protein according to claim 1 or 2, wherein the cleavage site segment has a protease cleavage site which is recognized and split specifically by the protease from human immunodeficiency virus, by the protease from the hepatitis C virus, by the protease from the tobacco etch virus, by the protease from human cytomegalovirus, by the protease from herpes simplex virus, by the protease plasmin, by the protease angiotensin converting enzyme, by the protease tPA of Factor Xₐ and/or thrombin.

4. Autofluorescent fusion protein according to one of the claims 1 to 3, wherein the first autofluorescent protein is the green fluorescent protein from Aequorea victoria (GFP) or a variant, particularly a red-shifted variant thereof (rsGFP), and the second autofluorescent protein is the dsRed from Discosoma sp. or a variant thereof.

5. Autofluorescent fusion protein according to one of the claims 1 to 4, which further includes other functional peptide sequences, in particular signal peptides, affinity marker peptides or detection marker peptides.

6. Autofluorescent fusion protein according to claim 1 which includes the sequence of fusion protein B or C (SEQ. ID. NOs:2 or 3).

7. A nucleic acid sequence which is coding for an autofluorescent fusion protein as defined in claims 1 to 6.

8. A vector, comprising a nucleic acid sequence as defined in claim 7.

9. A cell or non-human transgenic organism, comprising the nucleic acid sequence according to claim 7 and/or the vector according to claim 8.

10. A method for production of the autofluorescent fusion protein according to one of the claims 1 to 6, comprising the expression of the nucleic acid sequence according to claim 7 with the help of a cellular or cell-free expression system.

11. A method for analysis of a sample for proteolytic activity, comprising the steps:
(a) combining of the autofluorescent fusion protein as defined in claims 1 to 6 with the sample to be tested for proteolytic activity in an aqueous test solution;
(b) incubation under conditions which are suitable for proteolytic cleavage; and
(c) measurement of the proportion of split fusion protein by means of confocal fluorometric methods.

12. A method for analysis of a sample for protease inhibiting activity, comprising the steps:
(a) combining of the autofluorescent fusion protein as defined in the claims 1 to 6 with the sample to be tested for protease inhibiting activity and the appropriate protease in an aqueous test solution;
(b) incubation under conditions which are suitable for proteolytic cleavage; and
(c) measurement of the proportion of split fusion protein by means of confocal fluorometric methods.

13. A method for analysis of intracellular protease activity, comprising the steps:
(a) insertion of the nucleic acid sequence according to claim 7 and/or the vector according to claim 8 into the cell to be tested so that the autofluorescent fusion protein as defined in claims 1 to 6 is expressed intracellularly;
(b) incubation under conditions which are suitable for an expression and proteolytic cleavage of the fusion protein; and
(c) determination of the protease activity occurring intracellularly by means of confocal fluorometric methods.

14. A method for analysis of intracellular protease inhibiting activity, comprising the steps:
(a) insertion of the nucleic acid sequence according to claim 7 and/or the vector according to claim 8 into the cell to be tested so that the autofluorescent fusion protein as defined in claims 1 to 6 is expressed intracellularly;
(b) incubation under conditions which are suitable for an expression and proteolytic cleavage of the fusion protein; and
(c) determination of the protease inhibiting activity occurring intracellularly by means of confocal fluorometric methods.

15. A method according to one of the claims 11 to 14, whereby a dual-colour confocal fluorometric measurement method, in particular the dual-colour fluorescence cross-correlation spectroscopy (KK-FCS), the confocal fluorescence coincidence analysis (CFCA) or the 2D fluorescence intensity distribution analysis (2D-FIDA) is used as measurement method.

16. A method according to Claim 15, wherein the proportion of molecules displaying a fluorescence energy transfer is determined in addition to the dual-colour confocal fluorometric measurement methods.

17. Use of a method according to one of the claims 11 to 16 for the screening-based, evolutive optimization of biomolecules with proteolytic activity and/or the generation of biomolecules with proteolytic activity by screening-based directed evolution.

## Revendications

1. Protéine de fusion autofluorescente qui comporte une première protéine autafluorescente, un segment de liaison avec site de coupure par protéase, et au moins une autre protéine distinguable de la première, dans laquelle la fluorescence émise par les deux ou davantage des protéines autofluorescentes n'excite pas ou n'excite seulement que moins de 50 % de chacune des autres protéines autofluorescentes.

2. Protéine de fusion autofluorescente selon la revendication 1, où le segment de liaison avec site de coupure :
(i) est disposé entre deux polypeptides autofluorescents, distinguables l'un de l'autre par leurs propriétés spectrales et/ou
(ii) présente, outre le site de coupure par protéase, un peptide de liaison terminal, et/ou
(iii) présente une longueur d'au moins 20, de préférence plus de 20, plus particulièrement d'au moins 30 résidus d'acides aminés.

3. Protéine de fusion autofluorescente selon la revendication 1 ou 2, où le segment de liaison présente un site de coupure par protéase qui est spécifiquement reconnu et coupé par la protéase du virus de l'immunodéficience humain, par la protéase du virus de l'hépatite C, par la protéase du "tobacco Etch virus", par la protéase du cytomégalo-virus humain, par la protéase du Herpès Simplex Virus, par la protéase plasmine, par la Protease de conversion d'Angiotensine Enzyme, par la protéase tPa du facteur Xₐ et/ou par la thrombine.

4. Protéine de fusion autofluorescente selon l'une quelconque des revendications 1 à 3, dans laquelle la première protéine autofluorescente est une protéine à fluorescence verte provenant de Aequorea victoria (GFP) ou un de ses variants, en particulier un de ses variants décalés rouges (rsGFP) et où la seconde protéine autofluorescente est le dsRouge de Discosoma sp. ou un de ses variants.

5. Protéine de fusion autofluorescente selon l'une quelconque des revendications 1 à 4, qui présente en outre une séquence peptidique fonctionnelle, en particulier un peptide signal, un peptide marqueur d'affinité ou un peptide marqueur de détection.

6. Protéine de fusion autofluorescente selon la revendication 1, qui présente la séquence de protéine de fusion B ou C (SEQ ID N° 2 ou 3).

7. Séquence d'acide nucléique, qui code pour une protéine de fusion autofluorescente, telle que définie selon l'une quelconque des revendications 1 à 6.

8. Vecteur comprenant une séquence d'acide nucléique, telle que définie dans la revendication 7.

9. Cellule ou organisme transgénique non-humain, comportant la séquence d'acide nucléique selon la revendication 7 et/ou un vecteur selon la revendication 8.

10. Procédé de préparation d'une protéine de fusion autofluorescente selon l'une quelconque des revendications 1 à 6, comportant l'expression de la séquence d'acide nucléique selon la revendication 7 au moyen de systèmes d'expression cellulaires ou sans cellule.

11. Procédé d'analyse d'une sonde ayant une activité protéolytique consistant en les étapes suivantes :
- introduction de la protéine de fusion autofluorescente, telle que définie dans les revendications 1 à 6, avec une sonde à activité protéolytique à déterminer dans une solution d'essai aqueuse ;
- incubation dans les conditions qui conviennent à la coupure protéolytique ; et
- mesure de la fraction de protéine de fusion coupée au moyen de méthodes fluorométriques confocales.

12. Procédé d'analyse d'une sonde pour l'activité inhibitrice de protéase, comportant les étapes suivantes :
- introduction simultanée de la protéine de fusion autofluorescente telle que définie selon les revendications 1 à 6 avec la sonde dont on a étudié l'activité inhibitrice de protéase ainsi que la protéase correspondante dans une solution d'essai aqueuse.
- incubation dans des conditions qui conviennent à la coupure protéolytique et
- mesure de la fraction de la protéine de fusion dans une méthode fluorométrique confocale.

13. Procédé d'analyse de l'activité protéase intracellulaire, comportant les étapes suivantes :
- introduction de la séquence d'acide nucléique selon la revendication 7 et/ou des vecteurs selon la revendication 8 dans la cellule à étudier, de façon que la protéine de fusion autofluorescente selon l'une quelconque des revendications 1 à 6, soit exprimée intracellulairement ;
- incubation dans des conditions qui conviennent pour l'expression et pour la coupure protéolytique de la protéine de fusion ; et
- dosage de l'activité protéase présente intracellulairement au moyen de méthodes fluorométriques confocales.

14. Procédé d'analyse de l'activité inhibitrice de protéase comportant les étapes suivantes :
- introduction d'une séquence d'acide nucléique selon la revendication 7 et/ou des vecteurs selon la revendication 8 dans la cellule à étudier de manière que la protéine de fusion autofluorescente selon l'une quelconque des revendications 1 à 6, s'exprime intracellulairement ;
- incubation dans des conditions qui conviennent pour l'expression et une coupure protéolytique de la protéine de fusion ; et
- dosage de l'activité inhibitrice de protéase présente intracellulairement au moyen de méthodes fluorométriques confocales.

15. Procédé selon l'une quelconque des revendications 11 à 14, dans lequel en tant que méthode de mesure, on met en oeuvre une méthode de mesure fluorométrique confocale à deux couleurs, en particulier la spectroscopie de corrélation croisée de fluorescence bicolore (KK-FCS), une analyse de coïncidence de fluorescence confocale (CFCA) ou une analyse de répartition d'intensité de fluorescence 2D (2D-FIDA).

16. Procédé selon la revendication 15, dans laquelle, outre la méthode de mesure fluorométrique confocale bicolore, on détermine également la quantité d'une molécule présentant une fluorescence d'énergie de transfert.

17. Utilisation du procédé selon l'une quelconque des revendications 11 à 16, pour l'optimisation évolutive, basée sur un criblage de biomolécules ayant une activité protéolytique et/ou basée sur un criblage de biomolécules présentant une activité protéolytique.
